# DEMANDE DE BREVET EUROPEEN

(11) **EP 4 465 092 A1**
(43) Date de publication de la demande: **20.11.2024**
(21) Numéro de dépôt: 24175605.5
(22) Date de dépôt: 14.05.2024
(51) Int. Cl.: G01T 1/164, G01T 1/20, G21K 1/02, A61B 6/10, A61B 6/42, G01T 1/29, H04N 23/00

(54) **ENSEMBLE DE DETECTION DE RAYONNEMENT DIFFUSE ET SYSTEME DE DETECTION ET DE VISUALISATION COMPORTANT LEDIT ENSEMBLE DE DETECTION**

(30) Priorité: 17.05.2023 FR 2304954; 17.05.2023 FR 2304953
(71) Demandeur: Trixell, 38430 Moirans (FR)
(72) Inventeur: VIGNOLLE, Jean-Michel, 38430 MOIRANS (FR)
(74) Mandataire: Atout PI Laplace

(57) **Abrégé**

L'invention concerne un ensemble de détection de rayons diffusés comportant :
- un dispositif de filtrage (3) de rayons X configuré pour filtrer spatialement les rayons X en fonction de leur direction d'émission ; et
- un imageur (4) assemblé en vis-à-vis avec le dispositif de filtrage (3) de sorte à recevoir les rayons X filtrés par le dispositif de filtrage (3), l'imageur (4) étant configuré pour produire une image radiographique à partir des rayons X filtrés par le dispositif de filtrage (3) ;
le dispositif de filtrage (3) comprenant plusieurs lames, lesdites lames comprenant un matériau apte à absorber les rayons X, les lames étant orientées de sorte à s'écarter d'un centre du dispositif de filtrage (3) dans le sens allant de l'imageur (4) ves le dispositif de filtrage (4).

## Description

L'invention se rapporte à un ensemble de détection de rayonnement diffusé ainsi qu'à un système de détection et de visualisation comportant ledit ensemble de détection.

L'invention trouve son application dans divers domaines, par exemple dans l'imagerie par rayons X utilisée notamment dans le secteur médical.

Les rayons X émis suivant certaines directions peuvent se trouver au moins en partie diffusés selon d'autres directions non visées. Ainsi, les rayons X émis initialement en direction d'une ou plusieurs zones, se trouvent également sur d'autres zones non visées initialement. Ceci est le cas par exemple lors d'une radiologie interventionnelle dans laquelle un générateur de rayons X envoie des rayons X sur une zone du corps du patient. Une partie des rayons X dirigés vers le patient est absorbée par ce dernier et une autre partie de ces rayons X est diffusée par le patient ainsi que par les objets se trouvant à proximité et qui se trouvent touchés par ces rayons. Les rayons diffusés repartent dans toutes les directions. Le personnel médical est alors exposé à ce rayonnement diffusé, notamment lorsqu'il participe à l'intervention chirurgicale. Le rayonnement diffusé absorbé par le personnel médical au cours d'une intervention n'est pas dangereux, car la dose reçue est faible, mais l'exposition répétée au cours de multiples interventions peut être dangereuse.

Le personnel médical (et plus généralement tout utilisateur exposé aux rayons X) doit donc se protéger des rayons X. Pour ce faire, trois mesures principales de protection sont mises en oeuvre dans le secteur médical :
- port d'un dosimètre individuel, par exemple au niveau de la poitrine, le dosimètre donnant une indication approximative de la dose totale reçue par la personne au cours du temps ;
- port de vêtements de protection, par exemple en plomb, atténuant les rayons reçus ;
- le personnel médical est formé à ce risque et aux manières de le minimiser.

Ces mesures ne sont pas idéales. Le dosimètre, étant un petit objet, permet de mesurer le rayonnement reçu uniquement au niveau de la zone du corps sur laquelle il est fixé et ne rend donc pas compte du rayonnement reçu par d'autres parties du corps du personnel. Les vêtements de protection quant à eux sont lourds et inconfortables. Le personnel médical est donc réticent à les porter. Enfin, le personnel médical n'est pas toujours formé, comme expliqué dans l'article [Badera 2022], et même lorsqu'il est formé, étant donné que les rayons X sont invisibles, le personnel ne prend pas suffisamment en compte le danger.

Dans ce contexte, il peut être souhaitable de détecter les rayons X, notamment les rayons X diffusés. Un objectif peut être notamment de mieux se protéger des rayons X et/ou de protéger les équipements environnants. Il peut être également souhaitable de rendre les rayons X diffusés, visibles afin que, observant le danger, l'utilisateur puisse s'en protéger plus efficacement.

L'invention vise à proposer un ensemble de détection permettant de détecter les rayons X diffusés dans un environnement, ainsi qu'un système permettant de détecter et de visualiser les rayons X diffusés.

Elle propose à cet effet un ensemble de détection de rayons diffusés comportant :
- un dispositif de filtrage de rayons X configuré pour filtrer spatialement les rayons X en fonction de leur direction d'émission ; et
- un imageur assemblé en vis-à-vis avec le dispositif de filtrage de sorte à recevoir les rayons X filtrés par le dispositif de filtrage, l'imageur étant configuré pour produire une image radiographique à partir des rayons X filtrés par le dispositif de filtrage.
Le dispositif de filtrage comprend plusieurs lames, lesdites lames comprenant un matériau apte à absorber les rayons X.

Les lames comprennent une première série de lames et une deuxième série de lames, la première série de lames comprenant des premières lames se succédant selon une première direction, la deuxième série de lames comprenant des deuxièmes lames se succédant selon une deuxième direction. La première direction et la deuxième direction sont orthogonales entre elles.

Les lames sont orientées de sorte à s'écarter d'un centre du dispositif de filtrage dans le sens allant de l'imageur vers le dispositif de filtrage. Ainsi, les premières lames sont orientées de sorte à s'écarter du centre du dispositif de filtrage dans le sens allant de l'imageur vers le dispositif de filtrage, et les deuxièmes lames sont orientées de sorte à s'écarter du centre du dispositif de filtrage dans le sens allant de l'imageur vers le dispositif de filtrage.

Le dispositif de filtrage permet, par sa configuration en lames comprenant un matériau absorbant et ayant des orientations particulières, de former une image radiologique sur l'imageur qui ne voit que les rayons X arrivant selon certaines directions. Le dispositif de filtrage élimine (ou absorbe) les rayons X arrivant suivant une direction normale à un plan de l'imageur et laisse passer les rayons X selon d'autres directions qui arrivent ainsi sur l'imageur.

En outre, l'invention permet de détecter un rayonnement sur un champ relativement large. En effet, l'orientation des lames s'écartant du centre du dispositif de filtrage, i.e. vers l'extérieur du dispositif de filtrage, permet de détecter les rayons X sur un grand angle.

Des caractéristiques préférées particulièrement commodes de l'ensemble de détection selon l'invention sont présentées ci-dessous.

Au moins deux parmi les premières lames ne sont pas parallèles entre elles et au moins deux parmi les deuxièmes lames ne sont pas parallèles entre elles.

Des espaces séparent les lames entre elles.

Le dispositif de filtrage comprend des portions, dites portions transparentes, disposées entre les lames. Les portions transparentes sont réalisées en un matériau transparent aux rayons X.

L'imageur est courbe.

L'imageur présente une forme demi-sphérique.

L'imageur est plan.

Le dispositif de filtrage et l'imageur présentent une même forme générale.

Selon un autre aspect, l'invention concerne également un système de détection et de visualisation comportant :
- un ensemble de détection présentant au moins l'une des caractéristiques précédentes ;
- une caméra optique ayant un même champ de vision que l'imageur, la caméra optique étant configurée pour produire une image optique ; et
- un dispositif de lecture et d'affichage connecté à l'imageur et à la caméra, le dispositif de lecture et d'affichage étant configuré pour lire l'image reçue depuis l'imageur et l'image optique reçue depuis la caméra, superposer l'image radiographique et l'image optique, et afficher la superposition de l'image radiographique avec l'image optique.

La superposition de l'image X avec l'image optique permet de rendre les rayons X visibles et d'identifier leur localisation ainsi qu'éventuellement d'autres informations (intensité par exemple). Un observateur peut voir où se trouvent les zones émettrices de rayons X sur l'image optique.

La solution apportée par l'invention permet ainsi de visualiser les sources de rayons X diffusés susceptibles d'irradier un utilisateur tel qu'un personnel médical, ou aussi des rayons X arrivant sur un utilisateur et réémis par celui-ci, par exemple. Grâce au système de l'invention, la visualisation peut se faire en temps réel. Cela permet à l'utilisateur de se rendre compte sur le moment du danger. L'utilisateur ainsi sensibilisé prendrait alors les mesures de protection nécessaires.

Selon un autre aspect, l'invention peut concerner un procédé de détection et de visualisation de rayons X comprenant les étapes suivantes :
- filtrer des rayons X au moyen d'un dispositif de filtrage des rayons X ;
- produire une image, dite image X, à partir des rayons X filtrés par le dispositif de filtrage, l'image X étant produite au moyen d'un imageur de rayons X assemblé en vis-à-vis avec le dispositif de filtrage de sorte à recevoir les rayons X filtrés par le dispositif de filtrage ;
- produire une image optique à partir d'une caméra optique ayant un même champ de vision que l'imageur ;
- lire l'image X et l'image optique au moyen d'un dispositif de lecture et d'affichage connecté à l'imageur et à la caméra ;
- superposer l'image X et l'image optique au moyen du dispositif de lecture et d'affichage ; et
- afficher la superposition de l'image X avec l'image optique au moyen du dispositif de lecture et d'affichage.

Le dispositif de filtrage peut présenter au moins l'une des caractéristiques précédemment décrites.

D'autres particularités et avantages de l'invention apparaîtront encore dans la description ci-après en référence aux dessins annexés, donnés à titre d'exemples non limitatifs :
- la figure 1 représente schématiquement une salle de radiologie comprenant plusieurs systèmes de détection et de visualisation selon un mode de réalisation de l'invention ;
- la figure 2 représente individuellement le système de détection et de visualisation de la figure 1, ledit système comportant un ensemble de détection ;
- la figure 3 représente l'ensemble de détection avec une caméra optique ;
- la figure 4 représente un dispositif de filtrage de l'ensemble de détection selon un premier mode de réalisation ;
- la figure 5 représente le dispositif de filtrage selon un deuxième mode de réalisation ; et
- la figure 6 représente l'ensemble de détection selon un autre mode de réalisation de l'invention avec la caméra optique.

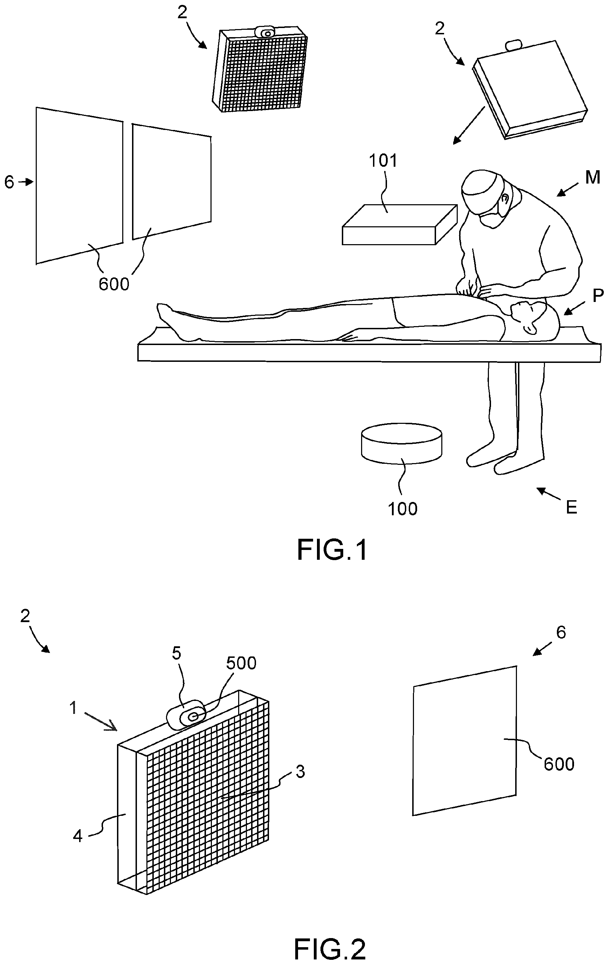

La figure 1 représente schématiquement une salle de radiologie dans laquelle se trouvent un patient P et un personnel médical M. La figure 1 illustre en outre un ensemble radiologique E dirigé vers le patient P. L'ensemble radiologique comporte un générateur 100 de rayons X et un détecteur de rayons X 101. Le patient P est placé entre le générateur 100 de rayons X et le détecteur 101.

Le générateur 100 de rayons X est configuré pour générer un faisceau de rayons X. Le générateur 100 de rayons X et le détecteur 101 sont positionnés l'un par rapport à l'autre, de façon à ce que les rayons X émis par le générateur 100 de rayons X soient captés par le détecteur 101.

Le détecteur 101 est un détecteur d'images traditionnel. Le détecteur d'images comprend notamment un capteur. Le capteur est par exemple réalisé sur un premier substrat. Le premier substrat comprend un ensemble de pixels organisé en matrice selon des lignes et des colonnes. La matrice peut comporter un nombre quelconque de lignes et de colonnes formant ainsi des pixels. La matrice forme une zone géographique sur le premier substrat. L'ensemble des pixels est configuré de façon à générer des signaux en fonction d'un rayonnement arrivant sur le détecteur 101.

Les pixels comprennent une zone photosensible délivrant un courant de charges électriques en fonction du flux de photons qu'elle reçoit, et un circuit électronique de traitement de ce courant. La zone photosensible comprend généralement un élément photosensible, ou photodétecteur, qui peut par exemple être une photodiode, une photorésistance ou un phototransistor. Le circuit électronique est constitué par exemple d'interrupteurs, capacités, résistances, en aval duquel est placé un actionneur. L'ensemble constitué par l'élément photosensible et le circuit électronique permet de générer des charges électriques et de les collecter.

La figure 1 représente en outre deux systèmes de détection et de visualisation 2 de rayons X selon un mode de réalisation de l'invention. Le système de détection et de visualisation 2 de rayons X selon cet exemple de réalisation est également représenté individuellement à la figure 2.

Le système de détection et de visualisation 2 comporte un ensemble de détection 1 de rayons X diffusés, une caméra optique 5 et un dispositif de lecture et d'affichage 6.

L'ensemble de détection 1 comporte un dispositif de filtrage 3 de rayons X et un imageur 4 de rayons X.

Les rayons X sont une forme de rayonnement électromagnétique à haute fréquence dont la longueur d'onde est comprise approximativement entre 5 picomètres et 10 nanomètres. L'énergie de ces photons va de quelques eV (électronvolt), à plusieurs dizaines de MeV. L'une des principales propriétés des rayons X est qu'ils pénètrent facilement « la matière molle » (matière solide peu dense et constituée d'éléments légers comme le carbone, l'oxygène et l'azote) et sont facilement absorbés par la « la matière dure » (matière solide dense constituée d'éléments lourds). Cette propriété permet l'imagerie médicale, les rayons traversant la chair et étant arrêtés par les os.

Le dispositif de filtrage 3 est configuré pour filtrer des rayons X.

Dans une situation de radiologie interventionnelle, les rayons X filtrés sont typiquement issus des rayons diffusés lorsqu'un patient reçoit des rayons par le générateur 100 de rayons X. En particulier, les rayons X filtrés sont issus des rayons diffusés « primaires », c'est-à-dire diffusés par le patient recevant des rayons émis par le générateur 100 de rayons X ainsi que par les objets se trouvant à proximité et qui se trouvent touchés par les rayons envoyés par le générateur 100 de rayons X. Les rayons X filtrés peuvent également être des rayons X diffusés « secondaires », c'est-à-dire issus de la diffusion par les objets ou le personnel a proximité, des rayons X émis par diffusion « primaire ».

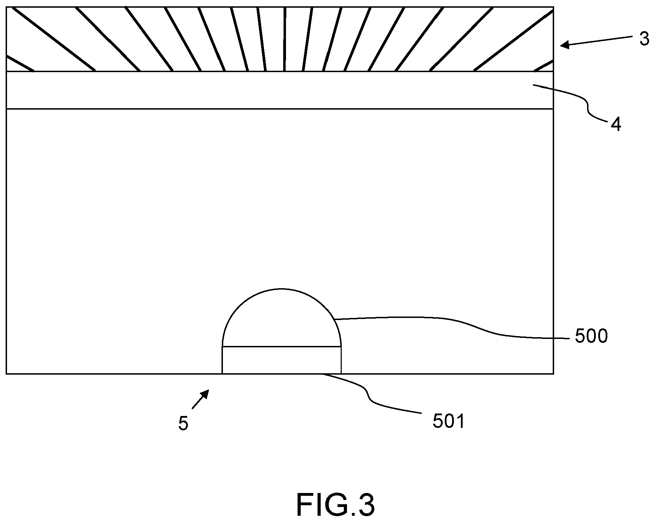

Le dispositif de filtrage 3 est assemblé en vis-à-vis avec l'imageur 4, comme représenté schématiquement sur les figures 1 à 3.

De préférence, la distance séparant le dispositif de filtrage 3 et l'imageur 4 est petite. De préférence, la distance séparant le dispositif de filtrage 3 et l'imageur 4 est inférieure à 30 cm. Cela permet une bonne détection des rayons X même lorsque leur flux est faible.

Le dispositif de filtrage 3 est ici fixé à l'imageur 4.

Le dispositif de filtrage 3 et l'imageur 4 présentent la même forme générale. La partie ou surface du dispositif de filtrage 3 assemblée à l'imageur 4 présente les mêmes dimensions que l'imageur 4. L'imageur 4 présente par exemple une forme générale rectangulaire. Le dispositif de filtrage 3 présente également des contours rectangulaires.

L'imageur 4 est plan dans le mode de réalisation des figures 1 à 3. Le dispositif de filtrage 3 est également plan. L'imageur 4 et le dispositif de filtrage 3 présentent sur la figure 1 une forme carrée. Par exemple, l'imageur 4 et le dispositif de filtrage 3 présentent chacun une forme carrée de 20 cm de côté.

Le dispositif de filtrage 3 et l'imageur 4 sont parallèles l'un à l'autre.

L'imageur 4 est configuré pour produire une image radiographique ou image X à partir des rayons X filtrés par le dispositif de filtrage 3.

L'imageur 4 est par exemple similaire au détecteur 101 de rayons X. L'imageur 4 comprend notamment un capteur. Le capteur est par exemple réalisé sur un premier substrat. Le premier substrat comprend un ensemble de pixels organisé en matrice selon des lignes et des colonnes. La matrice peut comporter un nombre quelconque de lignes et de colonnes formant ainsi des pixels. La matrice forme une zone géographique sur le premier substrat. L'ensemble des pixels est configuré de façon à générer des signaux en fonction d'un rayonnement arrivant sur l'imageur 4.

Les pixels sont sensibles aux rayons X et délivrent un signal électrique (notamment une charge électrique) dont le niveau est fonction de l'intensité des rayons X. Autrement dit, les pixels comprennent un élément photosensible, ou photodétecteur, qui peut par exemple être une photodiode, une photorésistance ou un phototransistor. Les signaux électriques issus des différents pixels sont collectés par des circuits de lecture de l'imageur 4 lors d'une phase de lecture de la matrice puis numérisés de manière à pouvoir être traités et stockés pour former l'image radiographique.

Les éléments photosensibles permettent de détecter un rayonnement électromagnétique visible ou proche du visible. Ces éléments ne sont pas, ou peu, sensibles au rayonnement incident au détecteur. On utilise alors fréquemment un convertisseur de rayonnement appelé scintillateur qui convertit le rayonnement incident, par exemple un rayonnement X, en un rayonnement dans une bande de longueurs d'onde auxquelles sont sensibles les éléments photosensibles présents dans les pixels. Une alternative consiste à réaliser l'élément photosensible dans un autre matériau réalisant la conversion directe du rayonnement X en charges électriques. C'est le cas par exemple des matrices dans lesquelles un premier substrat pixellisé en Tellurure de Cadmium (CdTe) est connecté pixel par pixel à un circuit de lecture CMOS qui ne possède donc plus la fonction de détection.

L'imageur 4 est de préférence très sensible et peu bruité. Cela s'exprime classiquement par la valeur de la NED du détecteur ("Noise Equivalent Dose", la dose dont le bruit d'X est égal au bruit électronique de l'imageur 4). Par exemple, le signal reçu par l'imageur 4 dans le cas d'une ouverture de 1 cm² à 20 cm², et d'une dose de rayons X envoyée sur le patient par une source de rayons X d'intensité 10mAs située à 1 m du patient, avec une énergie de 80kV à 120kV, pourrait être de seulement 0.01 à 0.05 nGy (nanoGray), ce qui est très peu. L'imageur 4 a donc la capacité de réaliser des images lisibles avec des doses de cet ordre de grandeur, et sa NED est typiquement inférieure ou égale à 0.01 nGy. Si la NED de l'imageur 4 est plus élevée, les images issues de l'imageur 4 sont moyennées dans le temps (moyennage temporel, par exemple de 100 images successives) et/ou, dans l'espace (moyennage spatial, par exemple par blocs de 10x10 pixels), ce qui limite la résolution temporelle et/ou spatiale, mais amène la NED au niveau désiré.

Le dispositif de filtrage 3 comprend un matériau apte à absorber les rayons X. Le matériau apte à absorber les rayons X est par exemple du plomb, du cuivre ou du tungstène. Le cuivre présente l'avantage d'être bon marché, rigide et ne présente pas de danger pour l'environnement.

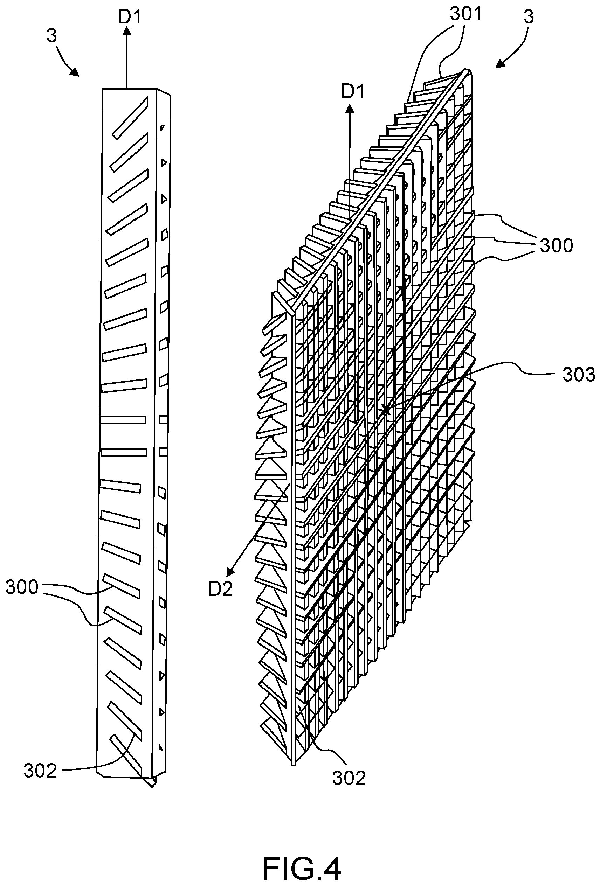

Le dispositif de filtrage 3 schématisé en figures 1 et 2 selon un mode de réalisation, est représenté individuellement et sous différents angles de vue en figure 4.

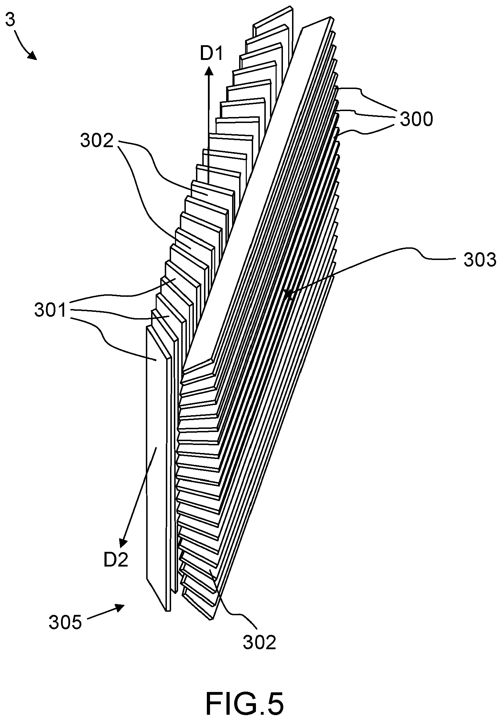

Le dispositif de filtrage 3 comprend plusieurs lames 300, 301 (représentés sur les figures 4, 5). Les autres figures représentent le dispositif de filtrage 3 de manière très schématique.

Les lames 300, 301 comprennent ou sont réalisées en un matériau apte à absorber les rayons X (par exemple du plomb, du cuivre ou du tungstène). Les lames 300, 301 sont orientées de sorte à absorber les rayons X selon plusieurs directions.

Chaque lame présente une forme rectangulaire. Chaque lame présente ainsi une dimension longitudinale ou longueur, une dimension latérale ou largeur, et une dimension transversale ou épaisseur.

Les lames 300, 301 sont ici séparées par des espaces 302. Autrement dit, un vide sépare entre elles les lames 300, 301 adjacentes.

Selon une variante de réalisation, les lames 300, 301 ne sont pas séparées par un espace vide. Le dispositif de filtrage 3 comprend des portions de matériau disposées entre les lames 300, 301. Les portions de matériau sont réalisées avec un matériau transparent aux rayons X. De préférence, les portions de matériau (dites aussi ici portions transparentes) sont rigides. Cela permet de maintenir fermement les lames 300, 301 en place. Les portions de matériau sont par exemple des portions de mousse en polyuréthane.

Les lames 300, 301 comprennent une première série de lames et une deuxième série de lames. La première série de lames comprend des premières lames 300 se succédant selon une première direction D1. La deuxième série de lames comprend des deuxièmes lames 301 se succédant selon une deuxième direction D2. La première direction D1 et la deuxième direction D2 sont distinctes, ici orthogonale entre elles.

Un espace 302 sépare ici chacune des premières lames 300. De même, un espace 302 sépare ici chacune des deuxièmes lames 301.

Les lames 300, 301 sont orientées de sorte à s'écarter d'un centre 303 du dispositif de filtrage 3. En particulier, les premières lames 300 sont orientées de sorte à s'écarter du centre 303 du dispositif de filtrage 3 et les deuxièmes lames 301 sont orientées de sorte à s'écarter du centre 303 du dispositif de filtrage 3. Les lames 300, 301 s'écartent du centre 303 dans le sens allant de l'imageur 4 vers le dispositif de filtrage 3. Autrement dit, les lames 300, 301 sont orientées vers l'extérieur du dispositif de filtrage 3. Les lames 300, 301 ainsi configurées, lorsque le dispositif de filtrage 3 est utilisé avec un ensemble radiologique, divergent d'un générateur de rayons X.

Les lames 300, 301 sont orientées de sorte à filtrer les rayons X selon plusieurs directions. Les lames 300, 301 sont orientées selon un angle d'orientation non nul par rapport au plan engendré par la première direction D1 et la deuxième direction D2.

Les lames 300, 301 sont chacune orientées selon un angle d'orientation prédéfini de sorte que, pour des lames 300, 301 données, les rayons X arrivant dans certaines directions, sont bloqués par lesdites lames 300, 301 données. Lesdites lames 300, 301 données absorbent les rayons X arrivant dans lesdites directions. Les rayons X arrivant selon une direction différente passent.

Lorsque le dispositif de filtrage 3 comprend des espaces 302 entre les lames 300, 301, les rayons X passent lorsqu'ils arrivent dans l'espace entre deux lames 300, 301. Autrement dit, les rayons X qui passent sont ceux qui n'arrivent pas sur une lame. En d'autres termes, les rayons X arrivant selon une direction donnée sont absorbés par les lames 300, 301 sauf dans une partie donnée du dispositif de filtrage 3 dans laquelle lesdits rayons X passent entre deux lames 300, 301 et se posent sur une zone donnée de l'imageur 4.

Le fonctionnement du dispositif de filtrage 3 est similaire lorsqu'il comprend un matériau transparent aux rayons X à la place des espaces 302 vides entre les lames 300, 301.

Grâce à cette configuration du dispositif de filtrage 3, chaque zone de l'imageur 4 ne voit qu'une direction de rayons X. La direction de rayons X vue par chaque zone de l'imageur 4 dépend au moins de l'orientation des lames 300, 301 du dispositif de filtrage 3.

L'angle d'orientation est modifié le long du dispositif de filtrage 3. Plusieurs lames 300, 301 peuvent avoir un même angle d'orientation. Les angles d'orientation des lames 300, 301 varient de sorte à absorber les rayons X selon certaines directions et en laisser passer selon d'autres directions.

De préférence, au moins deux parmi les premières lames 300 ne sont pas parallèles entre elles et au moins deux parmi les deuxièmes lames 301 ne sont pas parallèles entre elles. Autrement dit, au moins deux parmi les premières lames 300 ont des angles d'orientation différents l'un de l'autre et au moins deux parmi les deuxièmes lames 301 ont des angles d'orientation différents l'un de l'autre.

Le dispositif de filtrage 3 tel que décrit précédemment forme une lentille pour rayons X. Le dispositif de filtrage 3, par l'utilisation de plusieurs lames 300, 301 radio-opaques « sectionne » les rayons X. L'image radiographique finale obtenue par l'imageur 4 est un assemblage des images obtenues par la réception des rayons X « sectionnés » par le dispositif de filtrage 3. L'assemblage des images est effectué à la façon d'une mosaïque. L'assemblage des images est similaire à celui fait par les insectes ayant des yeux composés.

Le nombre de lames 300, 301, leur épaisseur et leur orientation définissent l'image filtrée. Le nombre de lames 300, 301, leur épaisseur et leur orientation sont prédéfinis et dimensionnés en fonction du besoin. Ces paramètres définissent l'écart (voire l'espace lorsqu'il y en a un) entre les lames 300, 301.

Les lames 300, 301 du dispositif de filtrage 3 ont une épaisseur comprise entre 0.1mm et 5mm, de préférence d'environ 1 mm.

La largueur de chaque lame 300, 301 est comprise entre 1mm et 10 cm, de préférence égale à 1 cm.

La longueur de chaque lame 300, 301 dépend de la taille de l'imageur 4 utilisé. La longueur de chaque lame est inférieure ou égale à la longueur de l'imageur 4, et de préférence égale à la longueur de l'imageur 4. La longueur de chaque lame est comprise entre 1cm et 50cm, de préférence égale à 20 cm.

L'angle d'orientation des lames 300, 301 est compris entre -90° et 90, de préférence entre -60° et 60°.

Le nombre de lames 300, 301 est compris entre 10 et 1000, de préférence d'environ 100.

Le dispositif de filtrage 3, plan dans les exemples de réalisation des figures 1 à 5, a une profondeur, i.e. une épaisseur totale, comprise entre 1 et 10 cm. Le dispositif de filtrage 3 est ainsi très compact.

L'image X est également fonction du nombre de pixels de l'imageur 4 se trouvant en vis-à-vis avec les espaces 302 entre les lames 300, 301. Plusieurs pixels sont de préférence en vis-à-vis avec chaque espace 302 entre les lames 300, 301.

Le dispositif de filtrage 3 peut être réalisé d'un seul tenant, comme cela est le cas sur la figure 4.

Dans une variante représentée à la figure 5, le dispositif de filtrage 3 est réalisé en deux parties. Le dispositif de filtrage 3 comprend une première partie 304 et une deuxième partie 305. La première partie 304 comprend la première série de premières lames 300. La deuxième partie 305 comprend la deuxième série de deuxièmes lames 301. Les lames sont chacune orientées selon un angle d'orientation prédéfini, similairement au mode de réalisation de la figure 4.

La première partie 304 et la deuxième partie 305 peuvent être superposées comme sur la figure 5.

Dans une variante non représentée, la première partie 304 et la deuxième partie 305 peuvent être entrecroisées.

La réalisation du dispositif de filtrage 3 en deux parties est plus économique que la réalisation en un seul tenant.

Le dispositif de filtrage 3 peut être fabriqué par impression 3D, par moulage, ou par tout autre moyen permettant d'obtenir la géométrie et les formes désirées.

Le dispositif de filtrage 3 est de préférence disposé à une distance de la zone à imager comprise entre 10 cm et 5m de préférence de l'ordre de 2m. Plus la distance est petite, meilleure est la qualité de l'image radiographique obtenue.

Cependant, il est à noter que l'imageur 4 présente l'avantage suivant. Le signal reçu par un pixel ne diminue pas avec la distance entre l'imageur 4 et la zone à imager, à condition que la zone à imager soit assez grande et couvre toujours le pixel. En effet, le signal de rayons X diminue en 1/R², R étant la distance entre l'imageur et la zone à imager, mais la surface de la zone qui est imagée dans un pixel croît comme R². Les deux effets se compensent, et le signal est constant.

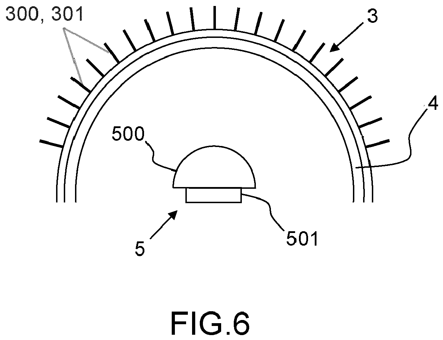

L'imageur 4 peut présenter une forme différente, par exemple l'imageur 4 peut être courbe. L'imageur 4 peut présenter une forme de dôme ou demi-sphère, comme illustré en figure 6. Le dispositif de filtrage 3 présente une forme similaire, ici de dôme.

L'imageur 4 et le dispositif de filtrage 3 présentent les mêmes caractéristiques que précédemment décrits pour l'imageur 4 et le dispositif de filtrage 3 plans.

En particulier, le dispositif de filtrage 3 comprend plusieurs lames 300, 301 s'écartant du centre 303 du dispositif de filtrage 3 dans le sens allant de l'imageur 4 vers le dispositif de filtrage 3.

Le dispositif de filtrage 3 est également ici fixé à l'imageur 4.

La forme en dôme ou demi-sphérique permet à l'imageur 4 de capter plus de rayonnement comparativement à la forme plane.

Le dôme peut typiquement présenter un diamètre compris entre 10 cm et 40 cm, par exemple 20 cm. La hauteur peut être comprise entre 5 cm et 20 cm, par exemple 10 cm.

L'image radiographique obtenue dépend comme expliqué ci-avant de la configuration du dispositif de filtrage 3. L'image radiographique est également fonction du nombre de pixels de l'imageur 4 se trouvant en vis-à-vis avec les espaces 302 entre les lames 300, 301 ou les portions en matériau transparent au rayonnement. Plusieurs pixels sont de préférence en vis-à-vis avec chaque espace entre les lames 300, 301, ou avec chaque portion en matériau transparent au rayonnement X.

Chaque pixel présente des dimensions de l'ordre de grandeur du millimètre, voire de quelques millimètres. Ces dimensions sont suffisantes pour les applications visées qui ne requièrent pas une haute résolution d'image.

La caméra optique 5, visible en figures 1, 2, 3 et 5, est configurée pour produire une image optique ou visible. La caméra 5 comprend une lentille 500 et un capteur photographique CCD ou CMOS 501.

L'imageur 4 et la lentille 500 de la caméra optique 5 sont dirigés vers un même point ou une même zone à capturer ou imager. La caméra optique 5 a sensiblement un même champ de vision que l'imageur 4. Par exemple, l'imageur 4 et la lentille 500 de la caméra 5 peuvent être dirigés vers les zones ou les personnes exposées aux rayons X.

La caméra optique 5 est disposée dans un plan parallèle à l'imageur 4. La caméra optique 5 peut être disposée au-dessus de l'imageur 4 (figure 1). La caméra optique 5 peut être disposée au centre ou point focal du dôme (figure 6) ou autre forme courbe.

Le dispositif de lecture et d'affichage 6 est connecté à l'imageur 4 et à la caméra 5. Le dispositif de lecture et d'affichage 6 est configuré pour lire l'image radiographique reçue depuis l'imageur 4 et l'image optique reçue depuis la caméra 5, superposer l'image radiographique et l'image optique, et afficher la superposition de l'image radiographique avec l'image optique.

Le dispositif de lecture et d'affichage 6 comprend des moyens de connexion à l'imageur 4 et à la caméra optique 5. Les moyens de connexion sont par exemple des connectiques ou des moyens de connexion sans fil.

Le dispositif de lecture et d'affichage 6 comprend un système de lecture et de traitement d'images. Le dispositif de lecture et d'affichage 6 peut comprendre un ordinateur pour le traitement d'image. Le traitement d'image peut être réalisé par un logiciel permettant la combinaison de l'image optique et de l'image radiographique.

Le dispositif de lecture et d'affichage 6 comprend en outre un afficheur 600, tel qu'un moniteur ou écran pour afficher la superposition de l'image radiographique et de l'image optique. L'afficheur 600 est connecté au système de lecture et de traitement d'images.

La superposition de l'image radiographique avec l'image optique montre les rayons X en les affichant sur l'image optique. Autrement dit, les rayons X diffusés peuvent être visualisés avec l'endroit où ils sont diffusés grâce à la superposition avec l'image optique.

Les rayons X peuvent être affichés dans une couleur différente de l'image optique. L'image optique peut être par exemple en noir et blanc, et les rayons X dans une autre couleur.

Des informations d'intérêt peuvent également être affichées, tel que l'intensité des rayons X. Les valeurs numériques de l'intensité peuvent être affichées et/ou les rayons X peuvent être affichés en différentes couleurs selon leur intensité.

Les zones diffusant les rayons X, ainsi qu'éventuellement d'autres informations tel que leur intensité, sont affichées en surimpression sur l'image optique. L'utilisateur peut voir cette information apparaître en temps réel sur l'afficheur 600. Cela pourrait par exemple l'inciter (dans le cas notamment du personnel médical) à se protéger des rayons X.

Un ou plusieurs systèmes de détection et de visualisation 2 peuvent être utilisés pour détecter et visualiser les zones diffusant des rayons X. Cela permet d'améliorer la détection et la visualisation des zones diffusant des rayons X. La figure 1 représente deux systèmes de détection et de visualisation 2.

Dans un exemple de réalisation, un afficheur 600 commun peut être utilisé pour plusieurs systèmes de détection et visualisation 2.

Le procédé de détection et de visualisation de rayons X se déroule de la manière suivante.

Les rayons X sont filtrés au moyen d'un dispositif de filtrage 3 des rayons X.

Les rayons X filtrés sont reçus par l'imageur 4. L'image X est produite par l'imageur 4 à partir des rayons X filtrés par le dispositif de filtrage 3.

En parallèle ou dans un autre temps, la caméra optique 5 produit une image optique.

L'image X et l'image optique sont toutes deux lues au moyen du dispositif de lecture et d'affichage 6 connecté à l'imageur 4 et à la caméra 5.

Le dispositif de lecture et d'affichage 6, au moyen par exemple d'un logiciel de traitement d'images, superpose l'image X et l'image optique.

Le dispositif de lecture et d'affichage 6 affiche enfin la superposition de l'image X et de l'image optique sur l'afficheur 4. Le dispositif de lecture et d'affichage 6 peut afficher d'autres informations tel que l'intensité des rayons X.

L'ensemble de détection selon l'invention permet par une structure et une configuration simples, de filtrer spatialement les rayons X en fonction de leur direction d'émission. Par sa configuration divergente du centre du dispositif de filtrage, celui-ci permet à l'ensemble de détection d'avoir un grand champ ou grand d'angle pour capter les rayons X sur l'imageur.

Le système de détection et de visualisation permet de détecter et de visualiser les zones de diffusion des rayons X de sorte à s'en protéger par exemple. L'affichage peut se faire en temps réel afin de fournir en direct l'information sur la diffusion des rayons X.

### Bibliographie

[Badera 2022] : Al Mohammad, Badera, Monther Gharaibeh, and Maram Al Alakhras. "Knowledge and practice of radiation protection in the operating theater among orthopédie surgeons." Journal of Medical Imaging 9.6 (2022): 066002.

## Revendications

1. Ensemble de détection de rayons diffusés comportant :
- un dispositif de filtrage (3) de rayons X configuré pour filtrer spatialement les rayons X en fonction de leur direction d'émission ; et
- un imageur (4) assemblé en vis-à-vis avec le dispositif de filtrage (3) de sorte à recevoir les rayons X filtrés par le dispositif de filtrage (3), l'imageur (4) étant configuré pour produire une image radiographique à partir des rayons X filtrés par le dispositif de filtrage (3),
le dispositif de filtrage (3) comprenant plusieurs lames (300, 301), lesdites lames (300, 301) comprenant un matériau apte à absorber les rayons X, les lames (300, 301) comprenant une première série de lames et une deuxième série de lames, la première série de lames comprenant des premières lames (300) se succédant selon une première direction (D1), la deuxième série de lames comprenant des deuxièmes lames (301) se succédant selon une deuxième direction (D2), la première direction (D1) et la deuxième direction (D2) étant orthogonale entre elles, les premières lames (300) étant orientées de sorte à s'écarter d'un centre (303) du dispositif de filtrage (3) dans le sens allant de l'imageur (4) vers le dispositif de filtrage (3), les deuxièmes lames (301) étant orientées de sorte à s'écarter du centre (303) du dispositif de filtrage (3) dans le sens allant de l'imageur (4) vers le dispositif de filtrage (3).

2. Ensemble de détection selon la revendication 1, dans lequel au moins deux parmi les premières lames (300) ne sont pas parallèles entre elles et au moins deux parmi les deuxièmes lames (301) ne sont pas parallèles entre elles.

3. Ensemble de détection selon la revendication 1 ou la revendication 2, dans lequel des espaces (302) séparent les lames (300, 301) entre elles.

4. Ensemble de détection selon la revendication 1 ou la revendication 2, dans lequel le dispositif de filtrage (3) comprend des portions, dites portions transparentes, disposées entre les lames (300, 301), les portions transparentes étant réalisées en un matériau transparent aux rayons X.

5. Ensemble de détection selon l'une des revendications 1 à 4, dans lequel l'imageur (4) est courbe.

6. Ensemble de détection selon l'une des revendications 1 à 5, dans lequel l'imageur (4) présente une forme demi-sphérique.

7. Ensemble de détection selon l'une des revendications 1 à 4, dans lequel l'imageur (4) est plan.

8. Ensemble de détection selon l'une des revendications 1 à 7, dans lequel le dispositif de filtrage (3) et l'imageur (4) présentent une même forme générale.

9. Système de détection et de visualisation comportant :
- un ensemble de détection (1) selon l'une des revendications 1 à 8 ;
- une caméra optique (5) ayant un même champ de vision que l'imageur (4), la caméra optique (5) étant configurée pour produire une image optique ; et
- un dispositif de lecture et d'affichage (6) connecté à l'imageur (4) et à la caméra (5), le dispositif de lecture et d'affichage (6) étant configuré pour lire l'image reçue depuis l'imageur (4) et l'image optique reçue depuis la caméra (5), superposer l'image radiographique et l'image optique, et afficher la superposition de l'image radiographique avec l'image optique.
